# EUROPEAN PATENT SPECIFICATION

(11) **EP 3 388 035 B1**
(45) Date of publication and mention of the grant of the patent: **12.04.2023**
(21) Application number: 16872448.2
(22) Date of filing: 09.12.2016
(51) Int. Cl.: A61F 5/05

(54) **VACUUM-MOLDED AND REUSABLE LIMB PROTECTING PAD**
VAKUUMGEFORMTES UND WIEDERVERWENDBARES GLIEDMASSENSCHUTZPAD
COUSSINET DE PROTECTION DE MEMBRE MOULÉ SOUS VIDE ET RÉUTILISABLE

(30) Priority: 09.12.2015 CN 201510901014; 09.12.2015 CN 201510903059
(43) Date of publication of application: 17.10.2018
(73) Proprietor: Chung, Ping-Chung, 300 Hsinchu City (TW); Selling-Ware Co., Ltd., Hsin-chu 302 (TW)
(72) Inventor: Chung, Ping-Chung, 300 Hsinchu City (TW)
(74) Representative: Lang, Christian
(86) International application number: PCT/CN2016/109301
(87) International publication number: WO 2017/097259

(56) References cited:
- EP-A2- 2 426 047
- CN-A- 101 229 085
- CN-U- 2 109 183
- CN-Y- 201 061 575
- CN-Y- 201 248 788
- US-A- 3 745 998
- US-A- 3 745 998
- US-A- 4 657 003
- US-A1- 2005 234 379
- US-A1- 2013 079 692

## Description

### BACKGROUND

### Field of the Invention

The present invention relates to a limb protecting pad, and more particularly to a vacuum-molded and reusable limb protecting pad, which can stably wrap around and abut against the surface of the limb (such as the head, the fractured part of the hand or foot, or the sprained neck or waist), by drawing air out of the limb protecting pad, the limb protecting pad will exert pressure to the granules filled therein, so that the granules are pressed against each other, and the limb protecting pad will be shaped into a pseudo solid state (its hardness or elasticity is determined by the amount of air drawn out) according to the contours of the limb, so as to protect the limb (such as absorption and mitigation of the impact of external force on the head, allowing the fractured hand or foot to be regenerated while being firmly positioned, or allowing the sprained neck and waist to get sufficient support).

### Related Prior Art

According to the World Health and Medical Organization (WHO), at least 10% of the world's population gets plastered every year because of broken limbs or surgery. Although "getting plastered" is a common but not particularly surprising thing, patients must still be psychologically prepared and willing to take the time to adapt and understand it so that they can get through the days of cast (plaster) treatment.

Generally speaking, doctors will choose the most suitable material according to the actual condition of the patient, it can be plaster cast made of gypsum powder or synthetic or resin cast. The plaster cast is made of gypsum powder and gauze, and the synthetic or resin cast is made of glass fiber and PU resin. However, no matter what kind of material is used, the wounded limbs usually continue to swelling during the first week of cast treatment to cause tightening of the cast, leading to poor blood circulation and pain. In the second week, when the swelling begins to recede, the patient will feel that the cast is too loose to hold the bone. So, at the very start, it takes a few days for the patient to get used to the cast, and the patient will always feel it a few days after putting on the cast. Besides, since the patient may or will never be able to do what they can do before the injury, he/she must slow down the life pace so as to slowly adjust and adapt to his/her own physical limitations, so that no negative effects on the recovery of affected parts will be caused during activity.

In addition, the first visit back is required within two weeks. However, if the patient is in the following conditions, he or she should visit back immediately so that the doctor can handle it properly after diagnosis:
(1) when the affected limb is in constant pain;
(2) when the patient feels that the cast is too tight and feel uncomfortable;
(3) when the cast gets loose gradually, or has cracks or rupture;
(4) when the pain of compression or friction is felt inside the cast;
(5) when the skin adjacent to the cast feels constant cold or turns pale; or
(6) when the finger or toe with cast feels pain, numbness, or persistent tingling.

Some time later, when the doctor diagnoses that the callus at the fracture site had been mature and completely recovered, a specially designed saw will be used to remove the cast from the affected limb. This saw is specially designed to cut the cast without hurting the soft skin, the process of cutting the cast is rapid and the patient feels no pain, however, the potential danger still exists, so that the patient must be able to fully cooperate with the doctor to minimize the risk.

According to the above, in the days of cast treatment, in addition to the aforementioned problems and many discomforts caused by cast to the affected limbs, after the completion of treatment, a large number of discarded casts after treatment also become a major burden on medical waste and causes a great waste of resources.

From document US 3 745 998 A a reusable limb protecting pad is known, which uses partition seams to directly join an upper sheet and lower sheet defining the airtight bag body, to subdivide a cavity into separate regions. Document EP 2 426 047 A2 also relates to a design of a reusable limb protecting pad, wherein separation means are provided for directly joining the opposite inner surfaces of an inner bag body together in order to divide an operating volume into a plurality of disconnected containing spaces to be filled with particles.

The problem to be solved by the invention is to design a limb protecting pad that can steadily surround and attach to the surface of the affected limb (e.g., the surface of a fractured hand or foot), and simply drawing the air out from the limb protecting pad can make the limb protecting pad exert pressure on a plurality of granules filled therein, so that the granules are pressed against each other, and the limb protecting pad will be shaped into a pseudo solid state (its hardness or elasticity is determined by the amount of air drawn out) according to the contours of the affected limb, thus allowing the fractured hand or foot to be regenerated while being firmly positioned. After the cast treatment is finished, air can be introduced into the limb protecting pad, that is, the granules filled in the limb protecting pad can be loosened and restored to a soft state, thereby enabling the patient to remove the limb protecting pad and to check, clean, apply medicine to the affected limb. Besides, the patient can readjust the shape of the limb protecting pad according to the contours of the newly recovered limb, so that the affected limb can continue to receive comfort, good and adequate support during rehabilitation until it is fully recovered.

The present invention has arisen to mitigate and/or obviate the afore-described disadvantages.

### SUMMARY

In view of the fact that the known plaster cannot be readjusted on the affected limb and cannot be reused, the inventor has, after many years of practical experience and many experiments and studies, has finally invented a vacuum-molded and reusable limb protecting pad, which can effectively solve many problems of the known plaster. It enables the user to reuse the limb protection pad during the treatment, the rehabilitation, or the protection of the affected limb. By adjusting the shape of the limb protecting pad, the limb can get the best treatment, rehabilitation and protection.

One objective of the present invention is to provide a vacuum-molded and reusable limb protecting pad comprising an airtight bag body, a nozzle, a plurality of granules, a filter screen and at least one tightening component. The airtight bag body is made of plasticized air impermeable material, a peripheral edge of the airtight bag body is sealed in an airtight manner to form a first containing space, an outer surface of the airtight bag body is provided with an opening at a position corresponding to the first containing space, the opening is in communication with the first containing space, a plurality of locating components are provided on two separated inner surfaces of the airtight bag body in a spaced manner to divide the first containing space into a plurality of locating spaces; the nozzle is airtight sealed to the airtight bag body in a position corresponding to the opening and provided with an air channel through which an air extractor is able to pump air out of the first containing space, so as to switch the air channel between an open state and a closed state. The plurality of granules is filled into the plurality of locating spaces. The filter screen is arranged at a position corresponding to the air channel, wherein the sieve size of the filter screen is less than particle size of the granules. The at least one tightening component (such as a strapping band or a Velcro strap) is capable of enabling the limb protecting pad to be wrapped around and abutted against a surface of a limb (such as the head, fractured hand or foot, or the sprained neck or waist), so as to adjust tightness of the limb protecting pad around the limb. Each of the plurality of locating components is formed by a positioning line having a specified length and is provided on two separated inner surfaces of the airtight bag body to divide the first containing space into a plurality of locating spaces which are spaced from but in communication with one another, so that a cross section of the first containing space is always maintained in a predetermined flat narrow shape.

Another objective of the invention is to provide a vacuum-molded and reusable limb protecting pad comprising: an inner bag, a plurality of granules, an outer bag, an air nozzle and at least one tightening component. The inner bag is made of breathable material, a peripheral edge of the inner bag is sealed to form a first containing space; a plurality of locating components are provided on two separated inner surfaces of the inner bag in a spaced manner to divide the first containing space into a plurality of locating spaces; the plurality of granules is filled into the locating spaces of the inner bag and each have a diameter larger than a diameter of vent apertures of the inner bag; the outer bag is made of air impermeable material, wherein a peripheral edge of the outer bag is airtight sealed to form a second containing space to accommodate the inner bag, an outer surface of the outer bag is provided with an opening aligned and in communication with the second containing space; the nozzle is airtight sealed to the outer bag in a position corresponding to the opening and provided with an air channel through which an air extractor (such as a vacuum cleaner) is able to pump air out of the first and second containing spaces; and the at least one tightening component (such as a strapping band or a Velcro strap) is capable of enabling the limb protecting pad to be wrapped around and abutted against a surface of a limb (such as the user's head, fractured hand or foot, or sprained neck or waist), so as to adjust tightness of the limb protecting pad around the limb. Each of the plurality of locating components is formed by a positioning line having a specified length and is connected to two separated inner surfaces of the inner bag to divide the first containing space into a plurality of locating spaces, which are spaced from each other but in communication with one another, so that a cross section of the first containing space is always maintained in a predetermined flat narrow shape.

By such arrangements, when the limb protecting pad is stably wrapped around and abutted against the limb, the user can evacuate the outer bag (or the airtight bag body) through the air channel by using an air extractor. At this moment, the inner bag (or the filter screen) can completely prevent the granules from being sucked out, only the air in the limb protecting pad will be drawn out by the air extractor, causing the outer bag (or the airtight bag body) to deform inward to consequently squeeze the granules filled therein, as a result, the granules are pressed against each other, and the limb protecting pad will be shaped into a pseudo solid state (its elasticity or hardness depends on the degree of vacuum) according to the contours of the limb to be protected, such as: absorption and mitigation of the impact of external force on the head, allowing the fractured hand or foot to be regenerated while being firmly positioned, or the sprained neck and waist can get sufficient support. When the user does not need to use the limb protecting pad, he/she just needs to introduce air into the outer bag (or the airtight bag body) to loosen the granules, so that the limb protecting pad can be restored to a soft state to facilitate storage and get ready for reuse for the next time.

These together with other objects of the invention, along with the various features of novelty which characterize the invention, are pointed out with particularity in the claims annexed to and forming a part of this disclosure. For a better understanding of the invention, its technical features, operating advantages and the specific objects, should refer to the accompanying drawings and descriptive matter in which there are illustrated preferred specific embodiments of the invention.

### BRIEF DESCRIPTION OF THE DRAWINGS

Fig. 1 shows that the limb protecting pad of the invention is worn on the limb;
Fig. 2 is an exploded cross sectional view of a limb protecting pad in accordance with a first embodiment of the invention; and
Fig. 3 is an exploded cross sectional view of a limb protecting pad in accordance with a second embodiment of the invention.

Description of the main components

| | | |
|---|---|---|
| Airtight bag body | | 10 |
| Filter screen | | 112 |
| Opening | | 11,74 |
| first containing space | | 13, 33 |
| locating space | | 131, 331 |
| Tightening component | | 15 |
| Velcro strap | | 16 |
| locating component | | 17, 35 |
| Air nozzle | | 20 |
| Nozzle seat | | 21 |
| Air channel | | 211 |
| Airtight valve | | 22 |
| Cover | | 23 |
| Inner bag | | 30 |
| Granules | | 40 |
| bag | | 50 |
| storage space | | 51 |
| Exposed hole | | 52 |
| coating | | 60 |
| Outer bag | | 70 |
| Second containing space | | 73 |
| Limb protecting pad | | A |
| Limb | | B |

### DETAILED DESCRIPTION

The present invention will be clearer from the following description when viewed together with the accompanying drawings, which show, for purpose of illustrations only, the preferred embodiment in accordance with the present invention.

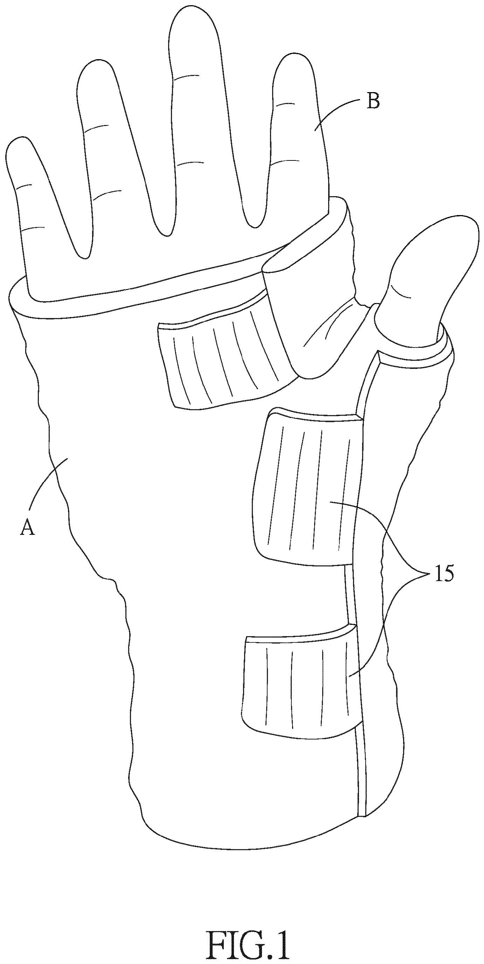

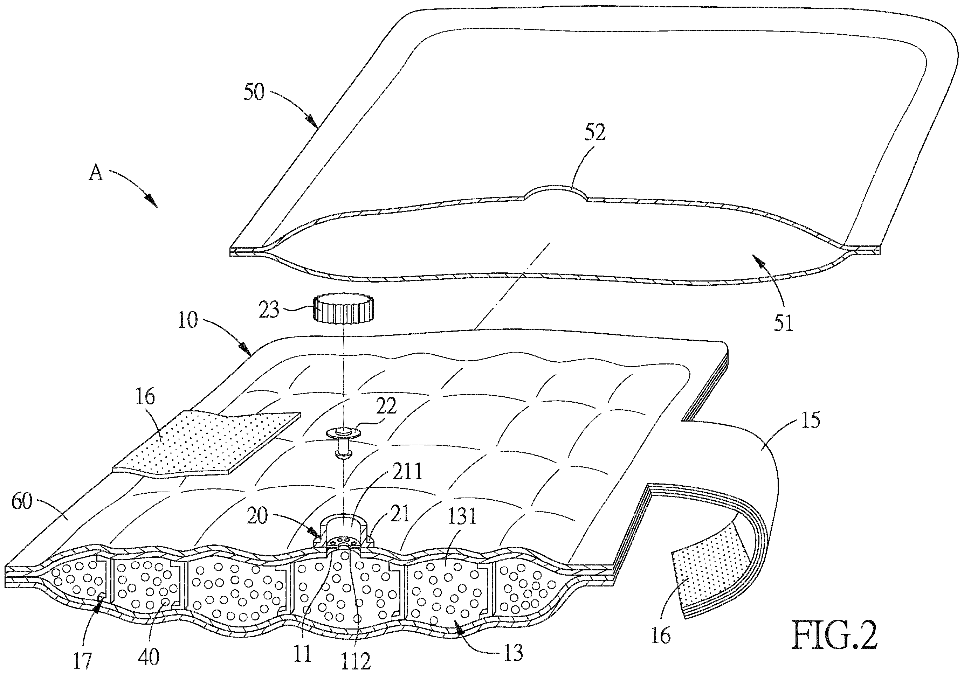

The invention relates to a vacuum-molded and reusable limb protecting pad A, as shown in Figs. 1 and 2, the limb protecting pad A comprises: an airtight bag body 10, an air nozzle 20 and a plurality of granules 40, a filter screen 112 and at least one tightening component 15. The airtight bag body 10 is made of plasticized air impermeable material, and the peripheral edge of the airtight bag body 10 is sealed in an airtight manner to form a first containing space 13. An outer surface of the airtight bag body 10 is provided with an opening 11 at a position corresponding to the first containing space 13, and the opening 11 is in communication with the first containing space 13. A plurality of locating components 17 are provided on two separated inner surfaces of the airtight bag body 10 in a spaced manner to divide the first containing space 13 into a plurality of locating spaces 131 which are spaced from but in communication with one another, so that the cross section of the first containing space 13 is always maintained in a predetermined flat narrow shape. In addition, the locating components 17 can be a wire, a belt, a tubular member, and other soft and hard connecting members or positioning points.

Referring to Figs. 1 and 2 again, in this first embodiment, the air nozzle 20 includes a nozzle seat 21 and a cover 23. The nozzle seat 21 is made of air impermeable material and has its lower peripheral edge sealed to an outer surface of the airtight bag body 10 in a position corresponding to the opening 11. The nozzle seat 21 is provided with an air channel 211 through which an air extractor (not shown) can pump air out of the first containing space 13. The cover 23 covers the top of the nozzle seat 21 to seal the air channel 211 in an airtight manner. The granules 40 are filled into and movable between the locating spaces 131 of the airtight bag body 10. The filter screen 112 is arranged at a position corresponding to the air channel 211, and the sieve size of the filter screen 112 is less than the particle size of the granules 40. The two ends of the tightening component 15 (e.g., the strapping band) can be securely attached to corresponding portions of the limb protecting pad A, so that the limb protecting pad A can be stably wrapped around and abutted against the surface of a limb B, and the tightness of the limb protecting pad A around the limb B can be adjusted. When the limb protecting pad A is stably wrapped around and abutted against the limb B, the user can evacuate the airtight bag body 10 through the air channel 211 by using an air extractor. Since the sieve size of the filter screen 112 is less than the particle size of the granules 40, the granules 40 will be restricted within the airtight bag body 10, so that the limb protecting pad A can be shaped into a pseudo solid according to the contours of the affected limb. When the user does not need to use the limb protecting pad A, he/she just needs to introduce air into the airtight bag body 10 to loosen the granules 40 filled in the airtight bag body 10, so that the limb protecting pad A can be restored to a soft shape. In addition, the filter screen 112 can be arranged not only in the nozzle seat 21 at a position corresponding to the air channel 211, but also on the airtight bag body 10 at a position corresponding to the air channel 211 (or the opening 11), so that when the air extractor is drawing air out of the first containing space 13 through the air channel 211, the filter screen 112 can stop the granules 40 from being sucked out by the air extractor.

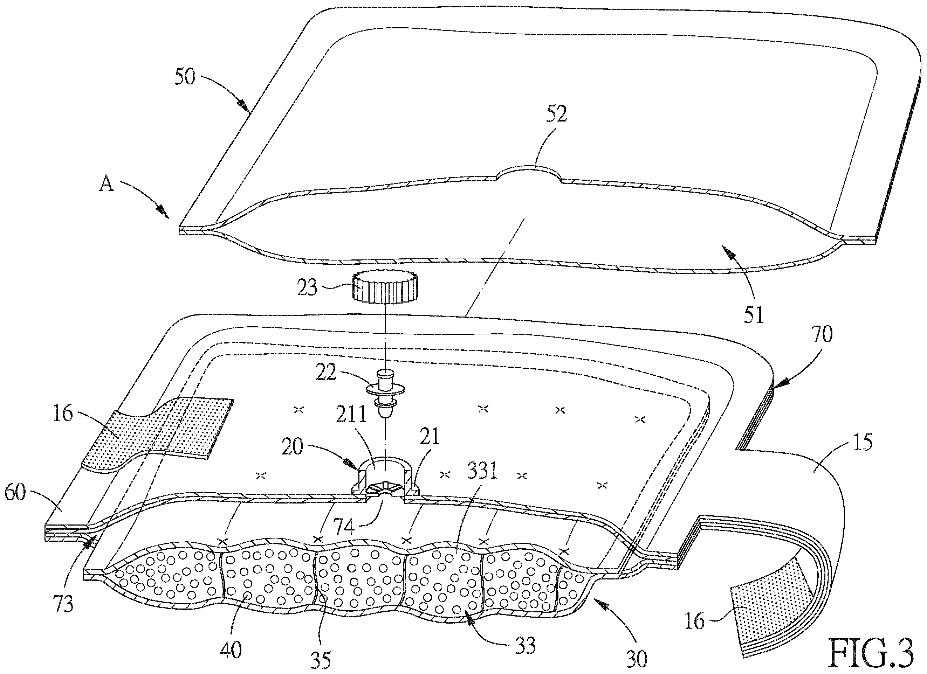

In addition, in a second embodiment of the invention, the single bag body is designed to include an outer bag and an inner bag to improve the convenience of assembly. As shown in Figs. 1 and 3, only the changed components in the following descriptions of the second embodiment are relabeled, but those with the same structure and general-purpose are still the same as the first embodiment. The limb protecting pad A comprises: an inner bag 30, an outer bag 70, at least one tightening component 15, air nozzle 20 and a plurality of granules 40. The inner bag 30 is made of a breathable material (e.g., a breathable fabric or a breathable plasticized film), the peripheral of the inner bag 30 is sealed to form a first containing space 33. A plurality of locating components 35 are provided on two separated inner surfaces of the inner bag 30 in a spaced manner to divide the first containing space 33 into a plurality of locating spaces 331 which are spaced from but in communication with one another, so that the cross section of the first containing space 33 is always maintained in a predetermined flat narrow shape. The granules 40 are movably filled in the locating spaces 331 of the inner bag 30 and each have a diameter larger than the diameter of the vent apertures of the inner bag 30. Thus, when the granules 40 are filled in the locating spaces 331 of the inner bag 30, the granules 40 may move between the locating spaces 331, but the locating components 35 keep the cross section of the first containing space 33 in a predetermined flat narrow shape, the granules 40 are more evenly dispersed in the locating space 331, so that the locating spaces 331 are maintained at a more uniform thickness rather than all stacked in one place. In addition, the granules 40 can only move between the locating spaces 331 without moving out of the inner bag 30 due to the fact that the diameter of the respective granules 40 is larger than the vent apertures of the inner bag 30. The foregoing is only a preferred embodiment of the present invention, the locating components 35 is not limited to a wire, but also can be a belt, a tubular member, and other soft and hard connecting members or positioning points.

Accordingly, if a skilled person in the art, after referring to the technical content of the present invention, replaces the locating components 35 with wires, belts, tubular members, other soft and hard connecting members, or positioning points (two inner surfaces of the inner bag are connected directly) with zero length, in order to make the locating spaces 331 spaced from but in communication with one another, and maintain the cross section of the first containing space 33 in a predetermined flat narrow shape; all these variations should not depart from the scope of the locating components 35 of the invention.

Referring to Figs. 1 and 3, the outer bag 70 is made of an impermeable material (e.g., an air impermeable plasticized film), the peripheral edge of the outer bag 70 is airtight sealed to form a second containing space 73 to accommodate the inner bag 30. An outer surface of the outer bag 70 is provided with an opening 74 aligned and in communication with the second containing space 73. The air nozzle 20 includes a nozzle seat 21 and a cover 23. The nozzle seat 21 is made of air impermeable material and has its lower peripheral edge sealed to a surface of the outer bag 70 in a position corresponding to the opening 74. The nozzle seat 21 is provided with an air channel 211 through which an air extractor (not shown) can pump air out of the second containing space 73 and the first containing space 33. The cover 23 covers the top of the nozzle seat 21 to seal the air channel 211 in an airtight manner. The tightening component 15 can be a strapping band with two ends securely attached to corresponding portions of the limb protecting pad A, so that the limb protecting pad A can be stably wrapped around and abutted against the surface of a limb B (such as head, the fracture part of the hands or feet, or the sprained part of the neck or the waist), the tightness of the limb protecting pad A around the limb B can be adjusted, and the granules 40 filled in the inner bag 30 can be positioned in the locating spaces 331 according to the contour of the limb B.

As shown in Figs. 1 and 3, when the limb protecting pad A is stably wrapped around and abutted against the limb B, the user can evacuate the outer bag 70 through the air channel 211 by using an air extractor (such as a vacuum cleaner). At this point, since the inner bag 30 can completely prevent the granules 40 from being sucked out, only the air in the first containing space 33 and the second containing space 73 will be drawn out by the air extractor, causing the outer bag 70 to deform inward to consequently squeeze the inner bag 30 and the granules 40 filled therein, so that the granules are pressed against each other, and the limb protecting pad will be shaped into a pseudo solid state (its elasticity or hardness depends on the degree of vacuum) according to the contours of the limb B, so as to protect the limb B, such as absorption and mitigation of the impact of external force on the head, thus allowing the fractured hand or foot to be regenerated while being firmly positioned, or the sprained neck and waist can get sufficient support. When the user does not need to use the limb protecting pad A, he/she just needs to introduce air into the outer bag 70 to loosen the granules 40 filled in the inner bag 30, so that the limb protecting pad A can be restored to a soft state to facilitate storage and get ready for reuse for the next time.

In another embodiment of the invention, as shown in Figs. 2 and 3, the air nozzle 20 can also be designed as a one-way reverse stop valve (i.e. check valve) or an air valve depending on actual requirements. The one-way reverse stop valve further includes an airtight valve 22 made of an impermeable material (e.g. an impermeable plasticized material) and can be movably disposed in the air channel 211. By such arrangements, in the first embodiment, when the extractor pump air out of the first containing space 13 via the air channel 211, the airtight valve 22 will open in this direction, whereas when the pressure outside the airtight bag body 10 is equal to or greater than the pressure within the first containing space 13, the airtight valve 22 will seal the air channel 211 automatically in an airtight manner. As to the air valve, user can open or close it according requirements, so that the air channel 211 can be switched between an open state and a closed state by user. Similarly, in the second embodiment, when the extractor pump air out of the first containing space 33 and the second containing space 73 via the air channel 211, the airtight valve 22 will open in this direction, whereas when the pressure outside the outer bag 70 is equal to or greater than the pressure within the first containing space 33 and the second containing space 73, the airtight valve 22 will seal the air channel 211 automatically in an airtight manner. The air valve can be opened or closed by user, so that the air channel 211 can be switched between an open state and a closed state according user's requirements.

In another embodiment of the present invention, as shown in Figs. 2 and 3, the limb protecting pad A also includes a bag 50, which is also made of a breathable material (such as a breathable fabric or a breathable plasticized film), the peripheral edge of the bag 50 is sealed to form a storage space 51. In the first embodiment, the storage space 51 is used to accommodate the airtight bag body 10, and in the second embodiment, the storage space 51 is used to accommodate the outer bag 70. The outer surface of the bag 50 is provided with an exposed hole 52 at a position corresponding to the air nozzle 20, and the air nozzle 20 can be exposed outside the bag 50 through the exposed hole 52. Thus, since the bag 50 covers the airtight bag body 10 (or the outer bag 70) and is made of breathable material, when the limb protecting pad A is shaped into a pseudo solid according to the contour of the limb B to stably wrap around and abut against the surface of the limb B, the bag 50 can provide the limb B with a better ventilation and perspiration effect and effectively avoid the discomfort (such as muggy or prone to allergies) to the surface of the limb B caused by the impermeability (due to the air impermeable material) of the airtight bag body 10 (or the outer bag 70). In addition, in order for the limb protection pad A of the present invention to have a simpler structure and a faster process, the bag 50 may also be replaced by a coating 60. The coating 60 is also made of breathable material (e.g., breathable fabric or breathable plasticized film) and is coated on the outer surface of the airtight bag body 10 (or the outer bag 70) so that only the air nozzle 20 can be exposed outside the coating 60, and the effect of the bag 50 can also be achieved.

In another embodiment of the present invention, as shown in Figs. 2 and 3, in order to reduce the overall weight of the limb protecting pad A, the granules 40 may be in the form of porous structure, wherein the granules 40 may be porous granules sintered from ceramic material, so that, when the granules 40 are pressed against each other, and the limb protecting pad A is shaped into a pseudo solid based on the contour of the limb B, a better shaping effect (its hardness depends on the degree of vacuum) can be produced to allow the fractured limb B (hands or feet) to be regenerated while being firmly positioned. The granules 40 may also be porous granules made of plastic foamed material, so that, when the granules 40 are pressed against each other, and the limb protecting pad A is shaped into a pseudo solid based on the contour of the limb B, a stable elastic shaping effect (its elasticity depends on the degree of vacuum) can be obtained to protect the limb B, for example, absorb or reduce the impact of external force on the head or provide sufficient support for the sprained neck or waist.

It is also worth mentioning that, as shown in Figs. 2 and 3, in the second embodiment, the tightening component 15 (such as a strapping band) has one end connected to the periphery of the outer bag 70, the bag 50 or the coating 60. In the first embodiment, the tightening component 15 has one end connected to the periphery of the airtight bag body 10, the bag 50 or the coating 60. And, the tightening component 15 has another end fixed to a corresponding part of the outer bag 70 (or the airtight bag body 10), the bag 50 or the coating 60 by a velcro strap 16. When the present invention is actually implemented, it is not limited to this. Those skilled in the art, after considering the technical content of the present invention, replace the Velcro strap 16 with other fasteners; as long as the purpose thereof is to enable the limb protecting pad A to tightly wrap around and abut against the surface of the limb B, and to adjust the tightness of the limb protecting pad A wrapping around the limb B, all these variations and efforts should not depart from the scope of the tightening component 15 of the invention.

What needs to be specially stated here is, as shown in Figs. 1 and 2, during the shaping operation, the doctor usually desires that the limb protecting pad A can always be maintained in a substantially flat, narrow and soft state. Since the airtight bag body 10 is generally under atmospheric pressure during shaping process, the granules 40 can move freely within the airtight bag body 10. If there is no design of the locating components 17 in the airtight bag body 10, when the user picks up one end or a part of the limb protecting pad A, the rest part of the limb protecting pad A that is not held by the user will fall down due to gravity. At this time, the granules 40 nearby the hand-gripping portion will drop downwards, causing the lower part of the airtight bag body 10 to bulge due to the accumulation of the granules 40, losing the predetermined flat shape of the limb protection pad A, which will make the shaping operation extremely difficult. Therefore, in practice, there is a need to provide the locating components 17 in the limb protecting pad A to limit the bulging degree of the airtight bag body 10, so that the limb protection pad A always has a flat and narrow shape with a predetermined thickness and has a soft and smooth surface, in order to facilitate the shaping operation.

Please refer to Figs. 1 and 2 again, a positioning line is the simplest locating component 17, that is, one line connects the corresponding points on the two inner sides of the airtight bag body 10 to limit the separation distance between the two inner side surfaces. In the extreme case where the length of the positioning line is zero, that is, the corresponding points on both inner sides of the airtight bag body 10 are directly joined, and the corresponding points are called the positioning points here. In addition, the concept of positioning point is quite direct, and practical production can also be achieved. As shown in Fig. 3, the upper and lower layers of the fabric of the inner bag 30 are stacked, and directly stitched at the corresponding points by a sewing machine. In contrast, the practice of the positioning line with length is not feasible in the known knowledge, so no one has claimed so far, and there are no examples of the positioning line. The main reason is that it is extremely difficult to produce. Taking a 35*35 cm fabric inner bag 30 as an example, it is assumed that there is a positioning line every 10 mm interval, the positioning line length is 10 mm, and the size of the inner bag 30 is suitable for the fixation of the fracture of the wrist. The difficult in making the inner bag 30 is that it is difficult to connect the two ends of the positioning line to the corresponding points of the upper and lower layers, and so far, there are no simple and convenient tools or methods, the most direct way is by sewing. However, the relationship between the positioning line and the upper and lower layers is a three-dimensional structure and soft. In addition, the length of the positioning line is only 10 mm, only stitching the two ends of a single positioning line to the upper and lower layers alone is very difficult already, not to mention that the inner bag 30 has 34^{∗}34=1156 positioning lines (i.e., 2312 connecting points), which is impractical without special equipments or production methods. The advantage of the limb protecting pad A made by the positioning line method is far beyond the reach of those made without using the locating components 35 or using with the positioning points.

As shown in the following table, which is a comparison table of the limb protecting pads A, where the inner bag 30 produced by "no locating component", "positioning point" and "positioning line" respectively; and the positioning interval is 10^{∗}10 mm, wherein the symbol "X" represents the result "Poor", "Δ" represents the result "Ordinary", and "Ⓞ" represents the result "Excellent":

| | Handheld shaping maintained flat | Flatness | Average thickness | Strength uniformity (vacuum) | Softness (atmosphere) | Granule filling & mobility |
|---|---|---|---|---|---|---|
| Without locating component | X | X | Δ (No restriction but uneven) | X | ⊚ | ⊚ |
| Positioning point | ⊚ | Δ | X (Thickness, limited, < 3mm) | Δ (The strength at the positioning | ⊚ | X |
| | | | | point is zero) | | |
| Positioning line | ⊚ | ⊚ | ⊚ (No restriction) | ⊚ | ⊚ | ⊚ |

Therefore, the inventor actively studied, made use of the special loom, modified it and greatly changed the workmanship and the way that the upper layer cloth, the lower layer cloth and the positioning line were woven simultaneously, was finally successfully made the inner bag 30 with the positioning line.

While we have shown and described various embodiments in accordance with the present invention, it is clear to those skilled in the art that further embodiments may be made, all should not depart from the protecting scope of the claims of the present invention.

## Claims

1. A vacuum-molded and reusable limb protecting pad (A) comprising:
an airtight bag body (10) made of air impermeable material, wherein a peripheral edge of the airtight bag body (10) is sealed in an airtight manner to form a first containing space (13); an outer surface of the airtight bag body (10) being provided with an opening (11) at a position corresponding to the first containing space (13), wherein the opening (11) is in communication with the first containing space (13); wherein a plurality of locating components (17) are provided between two inner surfaces of the airtight bag body(10) to divide the first containing space (13) into a plurality of locating spaces (131);
a nozzle airtight sealed to the airtight bag body (10) in a position corresponding to the opening (11) and provided with an air channel (211) through which an air extractor is able to pump air out of the first containing space (13);
a plurality of granules (40) filled into the plurality of locating spaces (131);
a filter screen (112) arranged at a position corresponding to the air channel (211), wherein the sieve size of the filter screen (112) is less than particle size of the granules (40); and
at least one tightening component (15) capable of enabling the limb protecting pad (A) to be wrapped around and abutted against a surface of a limb, so as to adjust tightness of the limb protecting pad (A) around the limb (B); **characterized in that**
each of the plurality of locating components (17) is formed by a positioning line having a specified length and being provided on two separated inner surfaces of the airtight bag body (10) to divide the first containing space (13) into a plurality of locating spaces (131) which are spaced from but in communication with one another, so that a cross section of the first containing space (13) is always maintained in a predetermined flat narrow shape.

2. The limb protecting pad (A) as claimed in claim 1, wherein the granules (40) are porous granules.

3. The limb protecting pad (A) as claimed in claim 2, wherein the porous granules are made of plastic foamed material or porous ceramic material.

4. The limb protecting pad (A) as claimed in claim 3, wherein the air nozzle (20) is a one-way reverse stop valve; wherein, when the extractor pumps air out of the first containing space (13) via the air channel (211), the one-way reverse stop valve will make the air channel (211) open in this direction; wherein, when a pressure outside the airtight bag body (10) is equal to or greater than a pressure within the first containing space (13) , the one-way reverse stop valve will seal the air channel (211) in an airtight manner.

5. The limb protecting pad (A) as claimed in claim 3, wherein the air nozzle (20) is an air valve which is capable of being opened or closed to open or close the air channel (211).

6. The limb protecting pad (A) as claimed in claim 1, 2, 3, 4 or 5, wherein the at least one tightening component (15) has one end connected to a periphery of the airtight bag body (10).

7. The limb protecting pad (A) as claimed in claim 1, 2, 3, 4 or 5, further comprising a bag (50) made of a breathable material, a peripheral edge of the bag (50) is sealed to form a storage space (51), the storage space (51) is used to accommodate the airtight bag body (10), an outer surface of the bag (50) is provided with an exposed hole (52) at a position corresponding to the air nozzle (20), so that the air nozzle (20) is exposed outside the bag (50) through the exposed hole (52).

8. The limb protecting pad (A) as claimed in claim 7, wherein the at least one tightening component (15) has one end connected to a periphery of the bag (50).

9. The limb protecting pad (A) as claimed in claim 1, 2, 3, 4 or 5, wherein a coating (60) made of breathable material is coated on the outer surface of the airtight bag body (10) and only the air nozzle (20) is exposed outside the coating (60).

10. The limb protecting pad (A) as claimed in claim 9, wherein the at least one tightening component (15) has one end connected to a periphery of the coating (60).

11. A vacuum-molded and reusable limb protecting pad (A) comprising:
an inner bag (30) made of breathable material, wherein a peripheral edge of the inner bag (30) is sealed to form a first containing space (33) ; wherein a plurality of locating components (35) are provided between two inner surfaces of the inner bag (30) to divide the first containing space (33) into a plurality of locating spaces (331);
a plurality of granules (40) filled into the locating spaces (331) of the inner bag (30) and each having a diameter larger than a diameter of vent apertures of the inner bag (30);
an outer bag (70) made of air impermeable material, wherein a peripheral edge of the outer bag (70) is airtight sealed to form a second containing space (73) to accommodate the inner bag (30); an outer surface of the outer bag (70) is provided with an opening (74) corresponding to and in communication with the second containing space (73);
a nozzle airtight sealed to the outer bag (70) in a position corresponding to the opening (74) and provided with an air channel (211) through which an air extractor is able to pump air out of the first and second containing spaces (33)(73); and
at least one tightening component (15) capable of enabling the limb protecting pad (A) to be wrapped around and abutted against a surface of a limb, so as to adjust tightness of the limb protecting pad (A) around the limb (B);
**characterized in that**
each of the plurality of locating components (35) is formed by a positioning line having a specified length and is connected to two separated inner surfaces of the inner bag (30) to divide the first containing space (33) into a plurality of locating spaces (331), which are spaced from each other but in communication with one another, so that a cross section of the first containing space (33) is always maintained in a predetermined flat narrow shape.

12. The limb protecting pad (A) as claimed in claim 11, wherein the breathable material is a breathable fabric or a breathable plasticized film.

13. The limb protecting pad (A) as claimed in claim 12, wherein the air impermeable material is an air impermeable plasticized film.

14. The limb protecting pad (A) as claimed in claim 13, wherein the granules (40) are porous granules.

15. The limb protecting pad (A) as claimed in claim 14, wherein the air nozzle (20) is a one-way reverse stop valve; wherein, when the extractor pumps air out of the first and second containing spaces (33)(73) via the air channel (211), the one-way reverse stop valve will make the air channel (211) open in this direction; wherein, when a pressure outside the outer bag (70) is equal to or greater than a pressure within the first and second containing spaces (33)(73), the one-way reverse stop valve will seal the air channel (211) in an airtight manner.

16. The limb protecting pad (A) as claimed in claim 14, wherein the air nozzle (20) is an air valve which is capable of being opened or closed to open or close the air channel (211).

17. The limb protecting pad (A) as claimed in claim 11, 12, 13, 14, 15 or 16, wherein the at least one tightening component (15) is a strapping band and has one end connected to a periphery of the outer bag (70).

18. The limb protecting pad (A) as claimed in claim 11, 12, 13, 14, 15 or 16 further comprising a bag (50) made of a breathable material and formed with a storage space (51), the storage space (51) being used to accommodate the outer bag (70), wherein an outer surface of the bag (50) is provided with an exposed hole (52) at a position corresponding to the air nozzle (20), so that the air nozzle (20) is exposed outside the bag (50) through the exposed hole (52).

19. The limb protecting pad (A) as claimed in claim 18, wherein the at least one tightening component (15) is a strapping band and has one end connected to a periphery of the bag (50).

20. The limb protecting pad (A) as claimed in claim 11, 12, 13, 14, 15 or 16, wherein a coating (60) made of a breathable material is coated on the outer surface of the outer bag (70) and only the air nozzle (20) is exposed outside the coating (60).

21. The limb protecting pad (A) as claimed in claim 20, wherein the at least one tightening component (15) is a strapping band and has one end connected to a periphery of the coating (60).

## Patentansprüche

1. Vakuumgeformtes und wiederverwendbares Gliedmaßenschutzpolster (A), umfassend:
einen luftdichten Beutelkörper (10) aus luftundurchlässigem Material, wobei ein Umfangsrand des luftdichten Beutelkörpers (10) luftdicht versiegelt ist, um einen ersten Aufnahmeraum (13) zu bilden, wobei eine Außenfläche des luftdichten Beutelkörpers (10) mit einer Öffnung (11) an einer Position versehen ist, die dem ersten Aufnahmeraum (13) entspricht, wobei die Öffnung (11) mit dem ersten Aufnahmeraum (13) in Verbindung steht, wobei eine Vielzahl von Anordnungskomponenten (17) zwischen zwei Innenflächen des luftdichten Beutelkörpers (10) vorgesehen ist, um den ersten Aufnahmeraum (13) in eine Vielzahl von Anordnungsräumen (131) zu unterteilen,
eine Düse, die luftdicht mit dem luftdichten Beutelkörper (10) in einer der Öffnung (11) entsprechenden Position versiegelt ist und mit einem Luftkanal (211) versehen ist, durch den ein Luftabsauger in der Lage ist, Luft aus dem ersten Aufnahmeraum (13) herauszupumpen,
eine Vielzahl von Granulaten (40), die in die Vielzahl von Anordnungsräumen (131) gefüllt sind,
ein Filtersieb (112), das an einer Position angeordnet ist, die dem Luftkanal (211) entspricht, wobei die Siebgröße des Filtersiebs (112) geringer ist als die Partikelgröße des Granulats (40), und
mindestens eine Spannkomponente (15), die in der Lage ist, es zu ermöglichen, dass das Gliedmaßenschutzpolster (A) um eine Oberfläche einer Gliedmaße gewickelt wird und an dieser anliegt, um so die Dichtigkeit des Gliedmaßenschutzpolsters (A) um die Gliedmaße (B) herum einzustellen,
**dadurch gekennzeichnet, dass**
jede der Vielzahl von Anordnungskomponenten (17) durch eine Positionierungslinie gebildet wird, die eine bestimmte Länge hat und auf zwei getrennten Innenflächen des luftdichten Beutelkörpers (10) vorgesehen ist, um den ersten Aufnahmeraum (13) in eine Vielzahl von Anordnungsräumen (131) zu unterteilen, die voneinander beabstandet sind, aber miteinander in Verbindung stehen, so dass ein Querschnitt des ersten Aufnahmeraums (13) immer in einer vorbestimmten flachen, engen Form gehalten wird.

2. Gliedmaßenschutzpolster (A) nach Anspruch 1, wobei das Granulat (40) ein poröses Granulat ist.

3. Gliedmaßenschutzpolster (A) nach Anspruch 2, wobei das poröse Granulat aus geschäumtem Kunststoffmaterial oder porösem Keramikmaterial besteht.

4. Gliedmaßenschutzpolster (A) nach Anspruch 3, wobei die Luftdüse (20) ein Einweg-Rückschlagventil ist, wobei, wenn der Extraktor Luft aus dem ersten Aufnahmeraum (13) über den Luftkanal (211) pumpt, das Einweg-Rückschlagventil den Luftkanal (211) in dieser Richtung öffnet, wobei, wenn ein Druck außerhalb des luftdichten Beutelkörpers (10) gleich oder größer ist als ein Druck innerhalb des ersten Aufnahmeraums (13), das Einweg-Rückschlagventil den Luftkanal (211) luftdicht verschließt.

5. Gliedmaßenschutzpolster (A) nach Anspruch 3, wobei die Luftdüse (20) ein Luftventil ist, das geöffnet oder geschlossen werden kann, um den Luftkanal (211) zu öffnen oder zu schließen.

6. Gliedmaßenschutzpolster (A) nach Anspruch 1, 2, 3, 4 oder 5, wobei die mindestens eine Spannkomponente (15) ein Ende hat, das mit einem Umfang des luftdichten Beutelkörpers (10) verbunden ist.

7. Gliedmaßenschutzpolster (A) nach Anspruch 1, 2, 3, 4 oder 5, ferner umfassend einen Beutel (50) aus einem atmungsaktiven Material, wobei ein Umfangsrand des Beutels (50) versiegelt ist, um einen Aufbewahrungsraum (51) zu bilden, wobei der Aufbewahrungsraum (51) verwendet wird, um den luftdichten Beutelkörper (10) unterzubringen, eine Außenfläche des Beutels (50) mit einem freiliegenden Loch (52) an einer Position versehen ist, die der Luftdüse (20) entspricht, so dass die Luftdüse (20) außerhalb des Beutels (50) durch das freiliegende Loch (52) freiliegt.

8. Gliedmaßenschutzpolster (A) nach Anspruch 7, wobei die mindestens eine Spannkomponente (15) ein Ende hat, das mit einem Umfang des Beutels (50) verbunden ist.

9. Gliedmaßenschutzpolster (A) nach Anspruch 1, 2, 3, 4 oder 5, wobei eine Beschichtung (60) aus atmungsaktivem Material auf die Außenfläche des luftdichten Beutelkörpers (10) aufgebracht ist und nur die Luftdüse (20) außerhalb der Beschichtung (60) freiliegt.

10. Gliedmaßenschutzpolster (A) nach Anspruch 9, wobei die mindestens eine Spannkomponente (15) ein Ende hat, das mit einem Umfang der Beschichtung (60) verbunden ist.

11. Vakuumgeformtes und wiederverwendbares Gliedmaßenschutzpolster (A), umfassend:
einen Innenbeutel (30) aus atmungsaktivem Material, wobei ein Umfangsrand des Innenbeutels (30) versiegelt ist, um einen ersten Aufnahmeraum (33) zu bilden, wobei eine Vielzahl von Anordnungskomponenten (35) zwischen zwei Innenflächen des Innenbeutels (30) vorgesehen sind, um den ersten Aufnahmeraum (33) in eine Vielzahl von Anordnungsräumen (331) zu unterteilen,
eine Vielzahl von Granulaten (40), die in die Anordnungsräume (331) des Innenbeutels (30) gefüllt sind und jeweils einen Durchmesser aufweisen, der größer ist als ein Durchmesser von Entlüftungsöffnungen des Innenbeutels (30),
einen Außenbeutel (70), der aus luftundurchlässigem Material hergestellt ist, wobei ein Umfangsrand des Außenbeutels (70) luftdicht versiegelt ist, um einen zweiten Aufnahmeraum (73) zu bilden, um den Innenbeutel (30) aufzunehmen, eine Außenfläche des Außenbeutels (70) mit einer Öffnung (74) versehen ist, die dem zweiten Aufnahmeraum (73) entspricht und mit diesem in Verbindung steht, eine Düse, die luftdicht mit dem Außenbeutel (70) in einer Position verbunden ist, die der Öffnung (74) entspricht, und die mit einem Luftkanal (211) versehen ist, durch den ein Luftabsauger in der Lage ist, Luft aus dem ersten und zweiten Aufnahmeraum (33) (73) herauszupumpen, und
mindestens eine Spannkomponente (15), die in der Lage ist, es zu ermöglichen, dass das Gliedmaßenschutzpolster (A) um eine Oberfläche einer Gliedmaße gewickelt wird und an dieser anliegt, um die Dichtigkeit des Gliedmaßenschutzpolsters (A) um die Gliedmaße (B) einzustellen,
**dadurch gekennzeichnet, dass**
jede der Vielzahl von Anordnungskomponenten (35) durch eine Positionierungslinie mit einer bestimmten Länge gebildet wird und mit zwei getrennten Innenflächen des Innenbeutels (30) verbunden ist, um den ersten Aufnahmeraum (33) in eine Vielzahl von Anordnungsräumen (331) zu unterteilen, die voneinander beabstandet sind, aber miteinander in Verbindung stehen, so dass ein Querschnitt des ersten Aufnahmeraums (33) immer in einer vorbestimmten flachen, schmalen Form erhalten wird.

12. Gliedmaßenschutzpolster (A) nach Anspruch 11, wobei das atmungsaktive Material ein atmungsaktives Gewebe oder eine atmungsaktive plastifizierte Folie ist.

13. Gliedmaßenschutzpolster (A) nach Anspruch 12, wobei das luftundurchlässige Material eine luftundurchlässige, plastifizierte Folie ist.

14. Gliedmaßenschutzpolster (A) nach Anspruch 13, wobei das Granulat (40) ein poröses Granulat ist.

15. Gliedmaßenschutzpolster (A) nach Anspruch 14, wobei die Luftdüse (20) ein Einweg-Rückschlagventil ist, wobei, wenn der Extraktor Luft aus dem ersten und dem zweiten Aufnahmeraum (33) (73) über den Luftkanal (211) pumpt, das Einweg-Rückschlagventil den Luftkanal (211) in dieser Richtung öffnet, wobei, wenn ein Druck außerhalb des Außenbeutels (70) gleich oder größer als ein Druck innerhalb des ersten und zweiten Aufnahmeraums (33) (73) ist, das Einweg-Rückschlagventil den Luftkanal (211) luftdicht verschließt.

16. Gliedmaßenschutzpolster (A) nach Anspruch 14, wobei die Luftdüse (20) ein Luftventil ist, das geöffnet oder geschlossen werden kann, um den Luftkanal (211) zu öffnen oder zu schließen.

17. Gliedmaßenschutzpolster (A) nach Anspruch 11, 12, 13, 14, 15 oder 16, wobei die mindestens eine Spannkomponente (15) ein Umreifungsband ist und ein Ende mit einem Umfang des Außenbeutels (70) verbunden ist.

18. Gliedmaßenschutzpolster (A) nach Anspruch 11, 12, 13, 14, 15 oder 16, ferner umfassend einem Beutel (50) aus einem atmungsaktiven Material, der einen Aufbewahrungsraum (51) aufweist, wobei der Aufbewahrungsraum (51) zur Aufnahme des Außenbeutels (70) genutzt wird, wobei eine Außenfläche des Beutels (50) mit einem freiliegenden Loch (52) an einer Position versehen ist, die der Luftdüse (20) entspricht, so dass die Luftdüse (20) außerhalb des Beutels (50) durch das freiliegende Loch (52) freiliegt.

19. Gliedmaßenschutzpolster (A) nach Anspruch 18, wobei die mindestens eine Spannkomponente (15) ein Umreifungsband ist und ein Ende mit einem Umfang des Beutels (50) verbunden ist.

20. Gliedmaßenschutzpolster (A) nach Anspruch 11, 12, 13, 14, 15 oder 16, wobei eine Beschichtung (60) aus einem atmungsaktiven Material auf die Außenfläche des Außenbeutels (70) aufgebracht ist und nur die Luftdüse (20) außerhalb der Beschichtung (60) freiliegt.

21. Gliedmaßenschutzpolster (A) nach Anspruch 20, wobei die mindestens eine Spannkomponente (15) ein Umreifungsband ist und ein Ende mit einem Umfang der Beschichtung (60) verbunden ist.

## Revendications

1. Coussinet de protection de membre moulé sous vide et réutilisable (A) comprenant :
un corps de sac étanche à l'air (10) constitué d'un matériau imperméable à l'air, dans lequel un bord périphérique du corps de sac étanche à l'air (10) est scellé de manière étanche à l'air pour former un premier espace de confinement (13) ; une surface extérieure du corps de sac étanche à l'air (10) étant munie d'une ouverture (11) dans une position correspondant au premier espace de confinement (13), dans lequel l'ouverture (11) est en communication avec le premier espace de confinement (13) ; dans lequel une pluralité de composants de localisation (17) sont prévus entre deux surfaces intérieures du corps de sac étanche à l'air (10) pour diviser le premier espace de confinement (13) en une pluralité d'espaces de localisation (131) ;
une buse scellée de manière étanche à l'air au corps de sac étanche à l'air (10) dans une position correspondant à l'ouverture (11) et munie d'un canal d'air (211) à travers lequel un extracteur d'air est capable de pomper de l'air hors du premier espace de confinement (13) ;
une pluralité de granules (40) remplissant la pluralité d'espaces de localisation (131) ;
un tamis filtrant (112) agencé dans une position correspondant au canal d'air (211), dans lequel la taille de maille du tamis filtrant (112) est inférieure à la taille de particule des granules (40) ; et
au moins un composant de serrage (15) capable de permettre au coussinet de protection de membre (A) d'être enroulé autour et mis en butée contre une surface d'un membre, de manière à ajuster un serrage du coussinet de protection de membre (A) autour du membre (B) ;
**caractérisé en ce que**
chacun de la pluralité de composants de localisation (17) est formé par une ligne de positionnement présentant une longueur spécifiée et étant prévue sur deux surfaces intérieures séparées du corps de sac étanche à l'air (10) pour diviser le premier espace de confinement (13) en une pluralité d'espaces de localisation (131) qui sont espacés mais en communication les uns avec les autres, de sorte qu'une section transversale du premier espace de confinement (13) est toujours maintenue dans une forme étroite et plate prédéterminée.

2. Coussinet de protection de membre (A) selon la revendication 1, dans lequel les granules (40) sont des granules poreuses.

3. Coussinet de protection de membre (A) selon la revendication 2, dans lequel les granules poreuses sont constituées d'un matériau en mousse plastique ou d'un matériau de céramique poreux.

4. Coussinet de protection de membre (A) selon la revendication 3, dans lequel la buse d'air (20) est un clapet anti-retour unidirectionnel ; dans lequel, lorsque l'extracteur pompe de l'air hors du premier espace de confinement (13) via le canal d'air (211), le clapet anti-retour unidirectionnel va amener le canal d'air (211) à s'ouvrir dans cette direction ; dans lequel, lorsqu'une pression à l'extérieur du corps de sac étanche à l'air (10) est égale ou supérieure à une pression à l'intérieur du premier espace de confinement (13), le clapet anti-retour unidirectionnel va sceller le canal d'air (211) de manière étanche à l'air.

5. Coussinet de protection de membre (A) selon la revendication 3, dans lequel la buse d'air (20) est une valve d'air qui peut être ouverte ou fermée pour ouvrir ou fermer le canal d'air (211).

6. Coussinet de protection de membre (A) selon la revendication 1, 2, 3, 4 ou 5, dans lequel le au moins un composant de serrage (15) présente une extrémité reliée à une périphérie du corps de sac étanche à l'air (10).

7. Coussinet de protection de membre (A) selon la revendication 1, 2, 3, 4 ou 5, comprenant en outre un sac (50) constitué d'un matériau respirant, un bord périphérique du sac (50) est scellé pour former un espace de stockage (51), l'espace de stockage (51) est utilisé pour accueillir le corps de sac étanche à l'air (10), une surface extérieure du sac (50) est pourvue d'un trou exposé (52) dans une position correspondant à la buse d'air (20), de sorte que la buse d'air (20) est exposée à l'extérieur du sac (50) à travers le trou exposé (52).

8. Coussinet de protection de membre (A) selon la revendication 7, dans lequel le au moins un composant de serrage (15) présente une extrémité reliée à une périphérie du sac (50).

9. Coussinet de protection de membre (A) selon la revendication 1, 2, 3, 4 ou 5, dans lequel un revêtement (60) constitué d'un matériau respirant est appliqué en revêtement sur la surface extérieure du corps de sac étanche à l'air (10) et seule la buse d'air (20) est exposée à l'extérieur du revêtement (60).

10. Coussinet de protection de membre (A) selon la revendication 9, dans lequel le au moins un composant de serrage (15) présente une extrémité reliée à une périphérie du revêtement (60).

11. Coussinet de protection de membre moulé sous vide et réutilisable (A) comprenant :
un sac intérieur (30) constitué d'un matériau respirant, dans lequel un bord périphérique du sac intérieur (30) est scellé pour former un premier espace de confinement (33) ; dans lequel une pluralité de composants de localisation (35) sont prévus entre deux surfaces intérieures du sac intérieur (30) pour diviser le premier espace de confinement (33) en une pluralité d'espaces de localisation (331) ;
une pluralité de granules (40) introduites dans les espaces de localisation (331) du sac intérieur (30) et chacune présentant un diamètre supérieur à un diamètre d'ouvertures de ventilation du sac intérieur (30) ;
un sac extérieur (70) constitué d'un matériau imperméable à l'air, dans lequel un bord périphérique du sac extérieur (70) est scellé de manière étanche à l'air pour former un second espace de confinement (73) destiné à accueillir le sac intérieur (30) ; une surface extérieure du sac extérieur (70) est munie d'une ouverture (74) correspondant à et en communication avec le second espace de confinement (73) ;
une buse scellée de manière étanche à l'air au sac extérieur (70) dans une position correspondant à l'ouverture (74) et munie d'un canal d'air (211) à travers lequel un extracteur d'air est capable de pomper de l'air hors des premier et second espaces de confinement (33)(73) ; et
au moins un composant de serrage (15) capable de permettre au coussinet de protection de membre (A) d'être enroulé autour et mis en butée contre une surface d'un membre, de manière à ajuster un serrage du coussinet de protection de membre (A) autour du membre (B) ;
**caractérisé en ce que**
chacun de la pluralité de composants de localisation (35) est formé par une ligne de positionnement présentant une longueur spécifiée et est relié à deux surfaces intérieures séparées du sac intérieur (30) pour diviser le premier espace de confinement (33) en une pluralité d'espaces de localisation (331), qui sont espacés les uns des autres mais en communication les uns avec les autres, de sorte qu'une section transversale du premier espace de confinement (33) est toujours maintenue dans une forme étroite et plate prédéterminée.

12. Coussinet de protection de membre (A) selon la revendication 11, dans lequel le matériau respirant est un tissu respirant ou un film plastifié respirant.

13. Coussinet de protection de membre (A) selon la revendication 12, dans lequel le matériau imperméable à l'air est un film plastifié imperméable à l'air.

14. Coussinet de protection de membre (A) selon la revendication 13, dans lequel les granules (40) sont des granules poreuses.

15. Coussinet de protection de membre (A) selon la revendication 14, dans lequel la buse d'air (20) est un clapet anti-retour unidirectionnel ; dans lequel, lorsque l'extracteur pompe de l'air hors des premier et second espaces de confinement (33)(73) via le canal d'air (211), le clapet anti-retour unidirectionnel va amener le canal d'air (211) à s'ouvrir dans cette direction ; dans lequel, lorsqu'une pression à l'extérieur du sac extérieur (70) est égale ou supérieure à une pression à l'intérieur des premier et second espaces de confinement (33)(73), le clapet anti-retour unidirectionnel va sceller le canal d'air (211) de manière étanche à l'air.

16. Coussinet de protection de membre (A) selon la revendication 14, dans lequel la buse d'air (20) est une valve d'air qui peut être ouverte ou fermée pour ouvrir ou fermer le canal d'air (211).

17. Coussinet de protection de membre (A) selon la revendication 11, 12, 13, 14, 15 ou 16, dans lequel le au moins un composant de serrage (15) est une bande de cerclage et présente une extrémité reliée à une périphérie du sac extérieur (70).

18. Coussinet de protection de membre (A) selon la revendication 11, 12, 13, 14, 15 ou 16 comprenant en outre un sac (50) constitué d'un matériau respirant et formé avec un espace de stockage (51), l'espace de stockage (51) étant utilisé pour accueillir le sac extérieur (70), dans lequel une surface extérieure du sac (50) est munie d'un trou exposé (52) dans une position correspondant à la buse d'air (20), de sorte que la buse d'air (20) est exposé à l'extérieur du sac (50) à travers le trou exposé (52).

19. Coussinet de protection de membre (A) selon la revendication 18, dans lequel le au moins un composant de serrage (15) est une bande de cerclage et présente une extrémité reliée à une périphérie du sac (50).

20. Coussinet de protection de membre (A) selon la revendication 11, 12, 13, 14, 15 ou 16, dans lequel un revêtement (60) constitué d'un matériau respirant est appliqué en revêtement sur la surface extérieure du sac extérieure (70) et seule la buse d'air (20) est exposée à l'extérieur du revêtement (60).

21. Coussinet de protection de membre (A) selon la revendication 20, dans lequel le au moins un composant de serrage (15) est une bande de cerclage et présente une extrémité reliée à une périphérie du revêtement (60).
